(19) Europäisches Patentamt
European Patent Office
Office européen des brevets

(11) Veröffentlichungsnummer: **0 334 218**
**A1**

(12) # EUROPÄISCHE PATENTANMELDUNG

(21) Anmeldenummer: 89104752.4

(22) Anmeldetag: 16.03.89

(51) Int. Cl.⁴: **A61K 31/245 , A61K 9/18**

(30) Priorität: 23.03.88 DE 3809808

(43) Veröffentlichungstag der Anmeldung:
27.09.89 Patentblatt 89/39

(84) Benannte Vertragsstaaten:
AT BE CH DE ES FR GB GR IT LI LU NL SE

(71) Anmelder: HEXAL-PHARMA GMBH & CO. KG
Industriestrasse 25
D-8150 Holzkirchen(DE)

(72) Erfinder: Horvath, Erich, Dr.
Prinz-Handjery-Strasse 22
D-1000 Berlin 37(DE)

(74) Vertreter: Wey, Hans-Heinrich, Dipl.-Ing.
Patentanwälte Wey & Partner
Widenmayerstrasse 49
D-8000 München 22(DE)

(54) **Feste, insbesondere festorale und rektale, Etofenamat enthaltende Arzneimittel.**

(57) Eine aus einer flüssigen, nicht komplexbildenden, an sich magen-und darmunverträglichen Wirkstoffkomponente, insbesondere Etofenamat, und Cyclodextrin bereitete feste Zubereitung stellt ein innerlich anwendbares festorales oder rektales, nun durchaus magen- und darmverträgliches Arzneimittel dar.

EP 0 334 218 A1

## Feste, insbesondere festorale und rektale, Etofenamat enthaltende Arzneimittel

Die Erfindung betrifft feste, insbesondere festorale und rektale Arzneimittel gemäß den vorstehenden Patentansprüchen, wie beispielsweise Tabletten, Dragees, Filmtabletten sowie Suppositorien.

Es ist bereits bekannt, daß flüssige, komplexbildende Wirkstoffe, z.B. etherische Öle, durch Komplexierung mit Cyclodextrinen in feste, mikrokristalline Komplexe umgewandelt werden können. Diese Komplexe weisen allgemein hohe Stabilität und gute Verpreßbarkeitseigenschaften zu festoralen Arzneiformen auf, da sie freifließend und nicht hygroskopisch sind. Die vollzogene Komplexbildung zwischen einem Wirkstoff und Cyclodextrinen ist im allgemeinen durch Methoden wie Röntgenkristallaufnahmen und Thermoanalyse nachweisbar.

Das unter der INN-Bezeichnung Etofenamat bekannte, analgetisch und antirheumatisch wirksame 2-(2-Hydroxyethoxy)-ethyl-N-($\alpha,\alpha,\alpha$-trifluoro-m-tolyl)-anthranilat der Formel

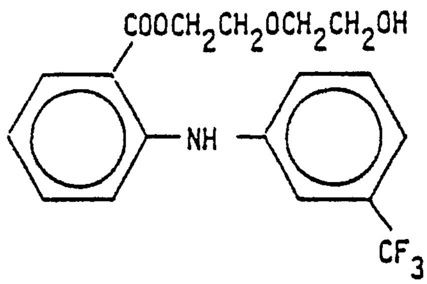

ist eine Flüssigkeit, und es reagiert mit einer Vielzahl von galenischen Hilfsstoffen, z.B. unter Hydrolyse, Veresterung u. dgl. Es findet allerdings bisher nur in äußerlich anwendbaren Arzneiformen, wie Salbe, Gel und Spray sowie in Form transdermaler Pflaster Anwendung. Seine Wirksamkeit nach oraler Applikation ist zwar beschrieben, aber es sind keine oralen Arzneiformen, wie bei anderen antirheumatischen Wirkstoffen üblich, im Handel, wofür es gewichtige Gründe gibt:

Bekanntlich werden flüssige Wirkstoffe als Weichgelatinekapseln zu peroralen Arzneiformen formuliert. In einer solchen Arzneiform ist im allgemeinen eine ausreichend gute Stabilität des Wirkstoffes gegeben. Im Falle der Applikation von Etofenamat als Weichgelatinekapsel verstärken sich jedoch die bekannten lokalen gastrointestinalen Unverträglichkeiten von nichtsteroidalen Antirheumatika noch erheblich durch eine spezifische Eigenschaft von Weichgelatinekapseln: Hat sich nämlich die Gelatine im Magensaft so weit gelöst, daß die Kapsel leckt, ergießt sich spontan der Kapselinhalt in den Magen. Dadurch auftretende lokale und hohe Wirkstoffkonzentrationen führen zu verstärkter Unverträglichkeit. Deswegen kann Etofenamat nicht als Weichgelatinekapsel formuliert werden.

Aufgabe der vorliegenden Erfindung war es deshalb, eine feste, d.h. festorale oder rektale Arzneiform anzugeben, die Etofenamat in wirksamer Menge enthält und dennoch magen- und darmverträglich ist. Diese Aufgabe wird durch das feste, d.h. festorale oder rektale, Arzneimittel gemäß den vorstehenden Patentansprüchen in vollem Umfang gelöst.

Überraschenderweise hat sich nämlich gezeigt, daß Etofenamat mit Cyclodextrinen in geeigneter Weise behandelt werden kann, wobei die dabei erhaltene feste Zubereitung entgegen allen Erwartungen kein üblicher Komplex ist, denn eine Änderung der Kristallstruktur von Cyclodextrinen ist nicht nachweisbar. Dennoch hat die erhaltene feste Zubereitung gemäß der Erfindung alle notwendigen und darüber hinaus vorteilhaften technologischen und pharmakologischen Eigenschaften, um festorale und rektale Arzneiformen herstellen zu können, die sehr gut verträglich und wirksam sind. Es ist durch die Erfindung somit erstmals möglich, Etofenamat nicht nur äußerlich, sondern ohne die sonst auftretenden Nebenwirkungen auch innerlich anwenden zu können.

Die erfindungsgemäßen festen Arzneimittel haben folgende technologische Eigenschaften:

- sie stellen ein freifließendes, nicht hygroskopisches Pulver mit guten Verpreßbarkeitseigenschaften dar;
- sie gehen keine Wechselwirkung mit den üblichen Tablettenhilfsstoffen ein, was die Stabilität des Wirkstoffes in der Arzneiform gewährleistet.

Darüber hinaus haben sie folgende pharmakologische Eigenschaften:

- im Magensaft ist die Benetzbarkeit verbessert und die Löslichkeit erhöht, so daß eine "Komplexbildung im gelösten Zustand" stattfindet;
- die Verträglichkeit ist durch erhöhte Resorption und geringe lokale Konzentrationen verbessert.

Das Etofenamat bildet somit mit Cyclodextrinen im festen Zustand nachweislich keinen Komplex, jedoch weist die erfindungsgemäße Zubereitung im festen Zustand die vorteilhaften Eigenschaften eines Komplexes auf. Dagegen bildet sich im gelösten Zustand offenkundig ein Komplex aus (Komplexbildung im gelösten Zustand ist nicht nachweisbar), woraus die verbesserte Magen-Darm-Verträglichkeit der erfindungsgemäßen Arzneimittel resultiert. Ihr Gehalt an Etofenamat richtet sich nach den jeweiligen Erfordernissen, beträgt jedoch vorzugsweise 7 bis 25 %.

Nachfolgend wird die Herstellung der erfindungsgemäßen Zubereitung und von eine solche enthaltenden Tabletten anhand eines Beispiels erläutert. Es folgen auch die Ergebnisse von Löslichkeits- und Stabilitätsuntersuchungen.

Beispiel

a) Herstellung einer Etofenamat-β-Cyclodextrinzubereitung:

140 g Etofenamat werden unter Rühren in ein Gemisch aus 1460 ml Wasser und 160 ml Ethanol gegeben. Daraufhin werden unter intensivem Rührem 860 g β-Cyclodextrin portionsweise zugefügt und die Suspension bei Zimmertemperatur weitere 24 Stunden gerührt. Danach darf kein freies Etofenamat (Ölabscheidung auf der Flüssigkeitsoberfläche) erkennbar sein.

Die Suspension wird filtriert, der Rückstand im Vakuumtrockenschrank bei 40° C getrocknet und durch ein Sieb (1 mm Maschenweite) zerkleinert.

Ausbeute: 850 g

Etofenamat-Gehalt (HPLC): 16,0 %

Aussehen: freifließendes, weißes Pulver

b) Herstellung von Etofenamat-β-Cyclodextrin-Tabletten:

Zusammensetzung je Tablette:

| | |
|---|---|
| Etofenamat-β-Cyclodextrinzubereitung (enthaltend 100 mg Etofenamat) | 625 mg |
| mikrokristalline Cellulose | 445 mg |
| Siliciumdioxid hochdispers | 20 mg |
| Palmitin-Stearinsäureglycerid | 10 mg |
| | 1100 mg |

Die Etofenamat-Zubereitung und die Hilfsstoffe werden in einem geeigneten Mischer homogenisiert und anschließend direkt zu Tabletten verpreßt.

Die vorstehend erwähnten günstigen Eigenschaften des erfindungsgemäßen Arzneimittels werden durch die nachfolgenden Versuchsergebnisse belegt.

Vergleich des Löslichkeitsverhaltens von reinem Etofenamat und zwei Etofenamat-Cyclodextrinzubereitungen gemäß der Erfindung:

Die Untersuchungen wurden in destilliertem Wasser bei 22° C durchgeführt und erbrachten die folgenden Ergebnisse:

| Untersuchte Probe | Etofenamat gelöst (mg/100 ml) | | | | | | | |
|---|---|---|---|---|---|---|---|---|
| | 1 | 3 | 5 | 10 | 15 | 30 | 45 | 60 (min) |
| Etofenamat | 0,9 | 1,1 | 1,1 | 1,1 | 1,3 | 1,3 | 1,1 | 1,2 |
| ß-Cyclodextrinzubereitung (Etofenamatgehalt 7,9 %) | 20,5 | 21,5 | 21,0 | 18,7 | 19,7 | 19,2 | 23,7 | 17,5 |
| ß-Cyclodextrinzubereitung (Etofenamatgehalt 16 %) | 19,7 | 18,7 | 16,0 | 16,0 | 16,2 | 16,0 | 16,5 | 16,5 |

Die Ergebnisse dieses Versuchs zeigen, daß die Löslichkeit des Etofenamats durch die erfindungsgemäße Formulierung erheblich gesteigert ist.

Vergleichende Stabilitätsuntersuchungen einer Etofenamat-ß-Cyclodextrinzubereitung gemäß der Erfindung und einer Etofenamat-Lactoseverreibung

Eine 16 %ige ß-Cyclodextrinzubereitung und eine ebenfalls 16 %ige Lactoseverreibung wurden auf den gleichen Wassergehalt (6,5 ± 0,3 %) eingestellt. Jeweils 5 Proben von ca. 2 g wurden in 5-ml-Ampullen eingeschmolzen und bei 40° C gelagert.

| Lagerzeit (Monate) | Gehalt Etofenamat (%) | |
|---|---|---|
| | ß-Cyclodextrinzubereitung | Lactoseverreibung |
| Ausgangswert: | 16,0 | 16,0 |
| 1 | 15,8 | 14,5 |
| 3 | 15,6 | 12,9 |
| 6 | 15,7 | 11,8 |

Es zeigt sich also, daß der Etofenamatgehalt bei der erfindungsgemäßen ß-Cyclodextrinzubereitung nach 6 Monaten nahezu unverändert geblieben war, während dieser Gehalt bei der Lactoseverreibung beträchtlich abgesunken war.

## Ansprüche

1. Festes Arzneimittel, enthaltend eine aus einer flüssigen, nicht komplexbildenden, magen- und darmunverträglichen Wirkstoffkomponente und Cyclodextrin bereitete feste Zubereitung neben den üblichen Hilfsstoffen.

2. Festorales Arzneimittel nach Anspruch 1, enthaltend eine aus Etofenamat und Cyclodextrin bereitete feste Zubereitung neben den üblichen Hilfsstoffen.

3. Rektales Arzneimittel nach Anspruch 1, enthaltend eine aus Etofenamat und Cyclodextrin bereitete feste Zubereitung neben den üblichen Hilfsstoffen.

4. Arzneimittel nach Anspruch 1, 2 oder 3, dadurch gekennzeichnet, daß ß-Cyclodextrin als Cyclodextrin Anwendung findet.

5. Arzneimittel nach einem der Ansprüche 1, 2, 3 oder 4, dadurch gekennzeichnet, daß es 7 bis 25 % Etofenamat enthält.

Europäisches Patentamt

# EUROPÄISCHER RECHERCHENBERICHT

Nummer der Anmeldung

EP 89 10 4752

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | US-A-4 073 931 (E. AKITO et al.)<br>* Ansprüche 1,5; Spalte 1, Zeilen 23-26; Spalte 2, Zeilen 12-18; Spalte 3, Zeilen 1-10 *<br>--- | 1-4 | A 61 K 31/245<br>A 61 K 9/18 |
| X,Y | GB-A-2 090 738 (CHINOIN et al.)<br>* Anspruch 1; Seite 1, Zeilen 45-50 *<br>--- | 1-4 | |
| Y | CHEMICAL ABSTRACTS, Band 90, Nr. 14, 2. April 1979, Seite 381, Zusammenfassung Nr. 109888e, Columbus, Ohio, US; N. NAMBU et al.: "Pharmaceutical interaction in dosage forms and processing. Part IX. Bioavailability of powdered inclusion compounds of nonsteroidal antiinflammatory drugs with beta-cyclodextrin in rabbits and dogs", & CHEM. PHARM. BULL. 1978, 26(10), 2952-6<br>* Zusammenfassung *<br>----- | 1-4 | |
| | | | RECHERCHIERTE SACHGEBIETE (Int. Cl.4) |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 16-06-1989 | SCARPONI U. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument